# EUROPEAN PATENT APPLICATION

(11) **EP 4 389 011 A1**
(43) Date of publication of application: **26.06.2024**
(21) Application number: 23175793.1
(22) Date of filing: 26.05.2023
(51) Int. Cl.: A61B 6/03, A61B 6/06, A61B 6/00

(54) **A COMPUTED TOMOGRAPHY SCANNING SYSTEM AND METHOD FOR PERFORMING A COMPUTED TOMOGRAPHY SCAN**

(71) Applicant: Siemens Healthineers AG, 91301 Forchheim (DE)
(72) Inventor: Baer-Beck, Matthias, 91080 Spardorf (DE); Hofmann, Christian, 91058 Erlangen (DE)
(74) Representative: Siemens Healthineers Patent Attorneys

(57) **Abstract**

A computed tomography scanning system (1) comprising a gantry (5) with an X-ray generating part (20) having at least one X-ray source (21) and an X-ray detecting part (30), wherein the X-ray generating part (20) and the X-ray detecting part (30) are rotatable around a scan region (11), wherein the X-ray generating part (20) is adapted such that it is capable of creating X-rays (23) via the at least one X-ray source (21) and sending the X-rays (23) into any part of the scan region (11) and wherein the X-ray detecting part (30) is configured to detect X-rays (23) generated by the X-ray generating part (20),wherein the X-ray generating part (20) is adapted to dynamically adjust the generated X-rays (23) such that at multiple rotational positions X-rays (23) are selectively sent into a partial region and/or such that X-rays (23) of different intensity are sent into different partial regions.

## Description

The invention relates to a computed tomography scanning system, a method for performing a computed tomography scan, and a corresponding computer program.

Independent of the grammatical term usage, individuals with male, female or other gender identities are included within the term.

Currently used third generation clinical Computed Tomography (CT) systems comprises a rotating part that is inside a gantry the of the CT system and adapted to rotate around a scan region where a patient may be placed. The rotating part usually comprises an X-ray source and at least one detector configured to detect the X-rays generated by the X-ray source. It is possible to adjust the scan range in the z-direction, i.e. the direction of movement of a patient table, which usually corresponds to the axial direction of the gantry. However, due to the setup of third generation CT systems, being based on a rotation of the X-ray source and detectors around the scan region, it is currently not possible to adjust the region that is scanned in the imaging plane of the CT system, i.e. the plane in x-y-direction, perpendicular to the z-direction.

One drawback of CT examinations is the radiation exposure of the patient during an examination. It is generally desirable to decrease the amount of radiation a patient is exposed to.

Furthermore, CT systems have made great progress when it comes to the achievable resolution, for example by providing detectors with small pixels sizes. This may lead to large amounts of data, especially when being combined with data heavy scan modes such as spectral CT imaging, wherein different X-ray spectra are acquired simultaneously or within relatively small timeframes. This may lead to the problem of the band width of the transmission path from the detector to an image reconstruction system being not sufficiently large to transmit all acquired data. Currently, this problem is sometimes addressed by only transmitting data of one spectrum in full resolution while data from the remaining spectra are down-sampled in order to save band width.

It is therefore an object of the invention to provide an improvement for at least one of the above-mentioned problems, in particular to provide a means with which the radiation dose on a patient may be reduced and/or that may help to improve on the amount of data acquired with data heavy scan modes such as high-resolution spectral scans.

This object is met or exceeded by a computed tomography scanning system according to claim 1, a method according to claim 12, and a computer program according to claim 15. Further features and advantages can be found in the dependent claims, the description, and the attached figures.

According to a first aspect of the invention, a computed tomography scanning system is provided. The computed tomography scanning system may preferably be a medical computed tomography scanning system. The computed tomography scanning system comprises a gantry with an X-ray detecting part and an X-ray generating part having at least one X-ray source. Therein, the X-ray generating part and the X-ray detecting part are rotatable around a scan region, the scan region being dividable into partial regions, wherein the X-ray generating part is adapted such that it is capable of creating X-rays via the at least one X-ray source and sending the X-rays into any part of the scan region and wherein the X-ray detecting part is configured to detect X-rays generated by the X-ray generating part, wherein the X-ray generating part is adapted to, during a scan in which the X-ray generating part is rotated around the scan region, dynamically adjust the generated X-rays such that, at multiple rotational positions, X-rays are selectively sent into a partial region and/or such that X-rays of different intensity are sent into different partial regions.

Advantageously, the system may thus provide the possibility to selectively illuminate a specific partial region. Hence it may be possible to reduce the radiation exposure of a subject, such as a patient, by only sending X-rays that are needed to illuminate the particular region or, alternatively, by sending X-rays with reduced intensity into one or multiple other regions. Hence, the system may enable to limit the region that is actually scanned in the imaging plane, i.e. the x-y-plane. Thus, it may advantageously be possible to not only change the scan range in z-direction, e.g. by moving a patient table, but also in the imaging range. CT systems of the third generation that are currently used are not capable of providing this possibility. Hence, for a given z-position, the full patient cross section is scanned and thus exposed to X-ray radiation by state-of-the-art CT systems, while the proposed system may allow to reduce the radiation exposure by fully exposing only a partial region.

The gantry may typically surround the scan region, in particular such that the gantry forms a short ring tunnel in which the scan region is located. Furthermore, the gantry may comprise a hollow ring-shaped interior in which the X-ray generating part and the X-ray detecting part can rotate around the scan region. Preferably, the X-ray generating part and the X-ray detecting part may be configured such that they rotate together around the scan region, in particular such that the X-ray detecting part is arranged on an opposite site of the ring-shaped form of the gantry. The X-ray detecting part may comprise a plurality of detector elements. Preferably the detector elements may be arranged in a semi-circle partially surrounding the scan region.

The scan region describes the region that can generally be reached by X-rays from the X-ray generating part during normal operation. Dividing the scan region into partial regions can be understood such that, within the scan region, various partial regions may be defined. The partial regions may be defined such that they correspond to the typical propagation of X-rays through the scan region. Hence, sending X-rays only into a partial region may thus mean that the partial region is shaped according to the propagation of these X-rays. On the other hand, the partial region may also be a region of interest that is defined based on the shape of an object, such as an organ or part of an organ, that is to be examined. "Dynamically adjust" the generated X-rays may in particular mean that the X-rays are adjusted during a scan, when the X-ray generating part is rotated. This may for example be advantageous when a region of interest is not in the center of the rotation. Hence, without dynamic adjustment, the X-rays would always be sent into the same direction relative to the rotated X-ray generating part but not necessarily into the same region of the non-moving scan region. Accordingly, the system may advantageously enable to adjust the direction of the X-rays according to a planned examination of a particular area, even if this area is not centered.

As will be explained in more detail, the inventive concept proposes two alternative ways of providing this feature, namely by using multiple X-ray sources that can be controlled individually, or by using a movable collimator that can be adjusted during a scan in order to selectively block X-rays. It may also be conceivable to combine these alternatives. For example, the system may be configured to dynamically control, in particular switch on and off, a plurality of X-ray sources and further adjust the X-rays generated by X-ray sources by moving the collimator. Sending X-rays of different intensity into different regions may be advantageous, for example, when more detailed information is needed from one main region and some but less detailed information is to be obtained from other regions, e.g. regions surrounding the main region or being adjacent to the main region.

According to an embodiment, the system is configured to receive a position of a region of interest that is a partial region within the scan region, wherein the system is configured to have a scan mode in which the X-ray generating part and the X-ray detecting part are rotated around the scan region and in which the X-ray generating part is configured to send only X-rays into the scan region that pass through the region of interest or is configured to send only first-type X-rays and second-type X-rays into the scan region, wherein the first-type X-rays pass through the region of interest, wherein the second-type X-rays do not pass through the region of interest and have a lower intensity than the first-type X-rays, in particular have no more than half the intensity of the first-type X-rays. Accordingly, this embodiment may be particularly advantageous, if only a relatively small region, i.e. the region of interest, needs to be fully examined. In this case, only the X-rays that are necessary to examine the region of interest may be sent to pass through a subject, such as a patient, and the total radiation exposure of the subject may be lower than in the case where a full scan of the full scan region is performed. Advantageously the system may thus be capable of selectively focusing on one of multiple regions of interest positions inside the scan region or inside the subject. Generally, covering only the region of interest may lead to truncation artifacts due to the sudden drop of signal at the border of the region of interest. However, it is usually possible to apply a data de-truncation method in order to reconstruct truncation artifact free images of the scan of the region of interest. For example, the data may be extrapolated outwards from the border of the region of interest in order to avoid truncation artifacts. Truncation is described in literature and various solutions for de-truncation are already known. For example, methods of de-truncation are described by B. Ohnesorge, T. Flohr, K. Schwarz, J. P. Heiken, and K. T. Bae, in "Efficient correction for CT image artifacts caused by objects extending outside the scan field of view", Medical Physics, 27(39), 2000 or by J. S. Maltz, S. Bose, H. P. Shukla, and A. R. Bani-Hashemi, in "CT Truncation artifact removal using waterequivalent thicknesses derived from truncated projection data", Proc. 29th Conf. of the IEEE EMBS, Lyon, France, 2007, or by A. A. Zamyatin and S. Nakanishi in "Extension of the reconstruction field of view and truncation correction using sinogram decomposition", Medical Physics, 34(5), 2007. According to one alternative it may be advantageous to also send X-rays with reduced intensity into other partial regions. The second-type X-rays may preferably be generated such that they cover the area around the region of interest. Data collected due to the second-type X-rays may be used during reconstruction of the images in order to avoid the occurrence of truncation artifacts. For example, if the reconstruction algorithm, that is used for reconstructing the image, is a filtered back projection type of algorithm the low dose projection data in the regions outside the region of interest may be used during the convolution step of the filtered back projection. Using the second-type x-rays may be done analogously to the method as described by Maass, Nicole, Knaup, Michael, Sawall, Stefan and Kachelrieß, Marc. in "Simple ROI Cone--Beam Computed Tomography", IEEE Nuclear Science Symposium conference record. Nuclear Science Symposium. 3161-3168. 10.1109/NSSMIC.2010.5874385, 2010.

According to an embodiment, the system is configured to perform at least a first scan protocol, in particular upon user activation, wherein the first scan protocol comprises performing a planning scan covering the whole scan region, prompting a user to choose the region of interest from a visualization of the planning scan's image data or automatically determining the region of interest, and consecutively performing a plurality of partial scans covering only part of the scan region while covering the region of interest. Advantageously, the planning scan may be used to determine the region of interest. Optionally, the system may be configured to perform the planning scan with a reduced dose level, in particular with a lower intensity of individual X-rays compared to the partial scans. This may be advantageous, in that the radiation exposure may be reduced since, at least in some instances, the scan quality of the planning scan does not have to be quite as good, e.g. just to provide an overview. The planning scan may be further used to plan other aspects of an examination. For example, the system may be applied for interventions that are monitored via the computed tomography system. The intervention may be planed based on the planning scan, e.g. by planning a needle path in the case of biopsy. Advantageously, the plurality of partial scans may, for example, be used as control scans during the intervention, such as to ensure that a needle follows a planned path and does not penetrate into organs along its way through a subject, in particular patient. Hence the flexibility of the system may be particularly advantageous in this or in other examples, in that a planning scan covering a wide range and the partial scans covering only a part of the planning scan's area may be executed consecutively. For the partial scans, the amount of radiation exposure may advantageously be reduced due to the smaller size of the region of interest with respect to the whole scan region, thus decreasing the disadvantage of needing multiple partial scans for monitoring or enabling to apply more partial scans, thus allowing a better monitoring. For example, the system, having multiple X-ray sources such as an array of X-ray sources, may be configured to activate all X-ray sources for the planning scan simultaneously and activate only some or only one of the X-ray sources during the partial scans simultaneously. In particular, the system may be configured to only activate the X-ray sources that are needed to cover and reconstruct the region of interest, e.g. the region where an intervention is currently to be observed. Alternatively or additionally, the X-rays that are not needed may be blocked by a moving collimator.

According to an embodiment, the X-ray detecting part comprises at least one photon counting detector, preferably a plurality of photon counting detectors, wherein the at least one photon counting detector is in particular configured to acquire X-ray data by differentiating between at least two X-ray energy levels of incoming X-rays by applying at least two energy thresholds. This embodiment may in particular be combined with any of the embodiments comprising the application of a region of interest. Advantageously, photon counting detectors are capable to acquire different X-ray spectra. Hence, data acquired by the photon counting detectors may be used to reconstruct spectral CT images, such as monoenergetic images, iodine maps or virtual non contrast images. Furthermore, photon counting detectors may be provided in particular small size, in particular smaller than classical scintillator-based X-ray detectors. This may be used to acquire data with small pixel sizes. Small pixel sizes may lead to an increased maximum spatial resolution. However, an increased maximum spatial resolution will usually also increase the amount of data that is generated and that needs to be transferred. In particular when combining high-resolution scans with the acquisition of spectral data, i.e. the acquisition of several spectra at once, the amount of data that needs to be transferred to an image reconstruction system may become too large for the available band width. Advantageously, by limiting the amount of X-ray data according to the region of interest, as described in the corresponding embodiments herein, the amount of data can advantageously be reduced by the limitation of a smaller region, i.e. the region of interest, thus allowing to acquire and transmit more spectral data in high resolution of this limited region than would be possible when scanning the full scan region.

According to an embodiment, the planning scan is a low-resolution scan, wherein the partial scans are high resolution scans each having a higher resolution than the planning scan and wherein the photon counting detector is configured to apply at least two thresholds for each partial scan and for the low-resolution scan. The planning scan, as described herein, may be used to reconstruct an overview image. The region of interest may be selected based on the overview image. Advantageously, the amount of data to be acquired and transferred may thus be reduced while still acquiring high-resolution data with at least two different spectra.

According to an embodiment, the X-ray generating part comprises a movable collimator in front of the at least one X-ray source, wherein the X-ray generating part is configured to dynamically move the collimator during a scan wherein the X-ray generating part is rotated around the scan region such that the X-rays are dynamically adjusted, in particular such that X-rays that would not pass through the partial region are blocked by the collimator. Preferably, the collimator is a fast-moving collimator. Moving the collimator may comprise moving a collimator opening of a collimator device. Advantageously, this embodiment may be implemented with only one X-ray source. Hence the dynamic adjustment of the X-rays may be achieved by blocking some X-rays from entering the scan region. Compared to the alternative of an array of X-ray sources, this embodiment may be relative low-cost, due to requiring only one X-ray source.

According to an embodiment, the X-ray generating part comprises an X-ray-source array with a plurality of X-ray sources including the at least one X-ray source, wherein the X-ray generating part is adapted to control the X-ray sources independently of each other. The X-ray sources may, for example, be distributed in a straight line. Alternatively, the X-ray sources may be distributed on the circumference of a partial circle, in particular a partial circle having its center point within the scan region. Advantageously, the system may be configured to focus on different, in particular arbitrarily chosen, regions of interest by controlling the X-ray sources individually. For example, the system may be configured to provide a user with the option to choose the region of interest.

Controlling the X-ray sources independently may in particular comprise switching the X-ray sources on and off individually and/or adjusting the energy of the X-ray radiation of the X-ray sources individually. In particular, the system may be configured to control the X-ray sources while the X-ray generating part rotates around the scan region. According to an embodiment, the X-ray generating part is adapted to switch individual X-ray sources on and off during a scan in which the X-ray generating part is rotated around the scan region such that only X-rays are sent into the scan region that pass through the partial region, in particular the region of interest. For example, for a given view angle, only those tubes may be switched on that are needed to cover the partial region, in particular the region of interest.

According to an embodiment, the X-ray sources of the source array are field-emitter-based X-ray tubes, in particular carbon nano tubes. Field-emitter-based X-ray tubes are advantageous, in that their relatively small size allows an efficient arrangement of these tubes on the array of X-ray sources. Field-emitter-based X-ray tubes provide the free electrons that are needed for the generation of X-rays via the effect of field emission. Hence, field-emitter-based X-ray tubes have the advantage over classical heat-based vacuum tubes of avoiding an effect of after glowing after having been switched off, since the provision of free electrons can be switched on and off more instantaneously. Carbon nano tubes can be particularly advantageous. For example, they can be switched particularly quickly.

According to an embodiment, the X-ray generating part is configured to dynamically change at least some of the pathways of the X-rays relative to the current position of the X-ray generating part during a rotation of the X-ray generating part. The pathways may be changed by changing the origin of the paths when entering the scan region. This may in particular be provided by blocking some X-rays and/or by switching some X-rays on or off. Alternatively or additionally, a collimator may be used to change the direction of the X-rays, e.g. blocking X-rays going in one particular direction. This may alternatively also be achieved by using at least two X-ray sources, each directing X-rays in different directions.

According to another aspect of the invention, an X-ray generating part for a gantry of a computed tomography scanner is provided. The X-ray generating part is rotatable around a scan region and comprises an X-ray-source array with a plurality of X-ray sources, the X-ray generating part being adapted such that it is capable of creating X-rays via the X-ray sources and sending the X-rays into any part of the scan region and wherein the X-ray generating part is adapted to control the X-ray sources independently of each other, in particular dynamically when the X-ray generating part is rotated around a scan region. All features and advantages of the system may be adapted to the X-ray generating part and vice versa. In particular the X-ray generating part may have the features and advantages of the X-ray generating part described herein in the context of the system. Advantageously, providing an X-ray generating part with a plurality of X-ray may allow to control the radiation exposure of a subject within the scan region by limiting the region X-rays are sent into and/or adjust the intensity of X-rays for different partial regions. Preferably, the X-ray sources may be field-emitter-based X-ray tubes, in particular carbon nano tubes, in particular as described herein.

According to a further aspect of the invention, a method for performing a computed tomography scan on a subject, in particular a patient, in a scan region with an X-ray generating part having at least one X-ray source and being rotatable around the scan region is provided. The method may in particular be carried out with a system or X-ray generating part as described herein. The method comprises the following steps:
(a) receiving or determining a position of a partial region within the scan region, in particular a region of interest;
(b) rotating the X-ray generating part around the scan region and sending only X-rays into the scan region that pass through the partial region, or
   sending first-type X-rays and second-type X-rays into the scan region, wherein the first-type X-rays pass through the partial region wherein the second-type X-rays do not pass through the partial region and have a lower intensity than the first-type X-rays, in particular have no more than half the intensity of the first-type X-rays;
(c) detecting the X-rays by an X-ray detecting part after they have passed the partial region.

The method and its embodiments may in particular be carried out as described in the context of the system. All features and advantages of the system and the X-ray generating part may be adapted to the method and its embodiments and vice versa.

According to an embodiment, the method includes the further steps of
- prior to step (a), performing a planning scan covering the whole scan region,
- during step (a), prompting a user to choose the region of interest from a visualization of the planning scan's image data or automatically determining the region of interest, and
- consecutively performing a plurality of partial scans covering only part of the scan region while covering the region of interest each partial scan comprising steps (b) and (c).

For example, it may thus be possible to use the planning scan in order to get an overview and/or plan the partial scans and then use the planning scans to get consecutive scans, e.g. to monitor time-dependent or dynamic events, while at the same time reducing the amount of radiation exposure of the subject.

According to an embodiment, the planning scan is a low-resolution scan, wherein the partial scans are high resolution scans each having a higher resolution than the planning scan, wherein the X-ray detecting part comprises photon counting detectors that apply at least two thresholds each for the partial scans and the low-resolution scan in order to differentiate between at least two X-ray energy levels. Additional steps of reconstructing an overview image based on data from the planning scan and selecting the region of interest based on the overview image may be added. Data acquired by the photon counting detectors may be used to reconstruct spectral CT images, such as monoenergetic images, iodine maps or virtual non contrast images. Advantageously, due to using partial scans, the amount of data to be acquired may thus be reduced while still acquiring high-resolution data with at least two different spectra.

According to an embodiment, when performing the second alternative of step (b) of using the first-type X-rays and second-type X-rays, the method may further comprise the following additional step:
(d) reconstructing a computed tomography image based on the data created via detecting the first-type X-rays and using the second-type X-rays to avoid the occurrence of truncation artifacts in the reconstructed image.

Advantageously, this embodiment may overcome the need for a truncation artifact correction. The low-dose data of the second-type X-rays from outside the region of interest may thus be used during the reconstruction. Based on the data from the second-type X-rays, the reconstruction itself may be performed according to known state-of-the-art reconstruction methods, such as described by Maass, Nicole, Knaup, Michael, Sawall, Stefan and Kachelrieß, Marc in "Simple ROI Cone--Beam Computed Tomography", IEEE Nuclear Science Symposium conference record. Nuclear Science Symposium. 3161-3168. 10.1109/NSSMIC.2010.5874385, 2010. The reconstruction algorithm may be a filtered back projection type of algorithm, in particular such that low dose projection data in the partial regions outside the region of interest can be used during the convolution step of the filtered back projection.

According to an embodiment the method may be adapted for performing a fluence field modulated computed tomography scan on the subject and comprise the following additional step:
- determining an intensity profile according to a predetermined attenuation profile of the subject;
wherein, in step (b), the X-rays sent into the scan region are modulated according to the intensity profile by controlling the intensity of X-rays from individual X-ray sources. Preferably, more than two different types of X-rays may be sent into the scan region, in particular such that different types of X-rays have different intensities. In fluence field modulated CT, the intensity profile of the X-ray beam is modulated and optimized to the patient's attenuation profile. The optimization may be performed separately for every view. In current state-of-the-art methods, the fluence filed modulation is typically performed by using a stationary wedge or bowtie filter that modulates the fluence field. However, in traditional fluence field modulated CT methods, due to the fact that the wedge filter is a stationary filter, the fluence field of the X-ray source cannot be adjusted from view to view but is fixed. Further, it cannot be changed or optimized to a given patient cross section but is a tradeoff that is designed to fit the overall average patient. Advantageously, by applying this embodiment, a means is provided to perform a dynamic fluence field modulation. All or selected X-ray sources may be operated simultaneously, wherein the intensity is adjusted. For example, the intensity may be adjusted by adjusting the tube current or the tube voltage of each X-ray source. The intensity may preferably be adjusted such that an overall dose distribution on the subject, in particular on a patient, is optimal for every projection acquired during a scan. The intensity at which the single tubes of the X-ray source array need to be operated to achieve an optimal dose distribution may be determined, for example, according to the method described in "Technical Note: Sheetbased dynamic beam attenuator - A novel concept for dynamic fluence field modulation in x-ray CT"; Sascha Manuel Huck, George S. K. Fung, Katia Parodi, Karl Stierstorfer and in Suess, C., Gies, M., Wolf, H., Madsen, M. T., & Kalender, W. A. (2002): "Dose reduction in CT by anatomically adapted tube current modulation. I. Simulation studies", Medical Physics, 26(11), 2235-2247. Furthermore, the methods described in in 8. Suess, C., Gies, M., Wolf, H., Madsen, M. T., & Kalender, W. A. (2002): "Dose reduction in CT by anatomically adapted tube current modulation. I. Simulation studies", Medical Physics, 26(11), 2235-2247 and in Kalender, W. A., Wolf, H., & Suess, C. (1999): "Dose reduction in CT by anatomically adapted tube current modulation. II. Phantom measurements", Medical Physics, 26(11), 2248-2253 may preferably be applied for each tube of the X-ray sources independently.

According to a further aspect of the invention, a computer program comprising instructions which, when the program is executed by a computed tomography system, in particular a system as described herein, cause the system to carry out the steps of the method as described herein. All features and advantages of the system, X-ray generating part, and the method may be adapted to the computer program and vice versa.

The embodiments described herein may be combined with each other unless indicated otherwise.

The accompanying drawings illustrate various exemplary embodiments and methods of various aspects of the invention.
- Fig. 1: shows a computed tomography (CT) scanning system with a gantry according to an embodiment of the invention;
- Fig. 2: shows a flow diagram of a method for performing a CT scan on a subject according to an embodiment of the invention;
- Fig. 3: shows a flow diagram of a method for performing a CT scan on a subject according to another embodiment of the invention;
- Fig. 4: shows a flow diagram of a method for performing a fluence field modulated computed tomography scan on a subject according to an embodiment of the invention;
- Fig. 5: shows several steps of the application of an X-ray generating part according to an embodiment of the invention; and
- Fig. 6: shows several steps of the application of an X-ray generating part according to another embodiment of the invention.

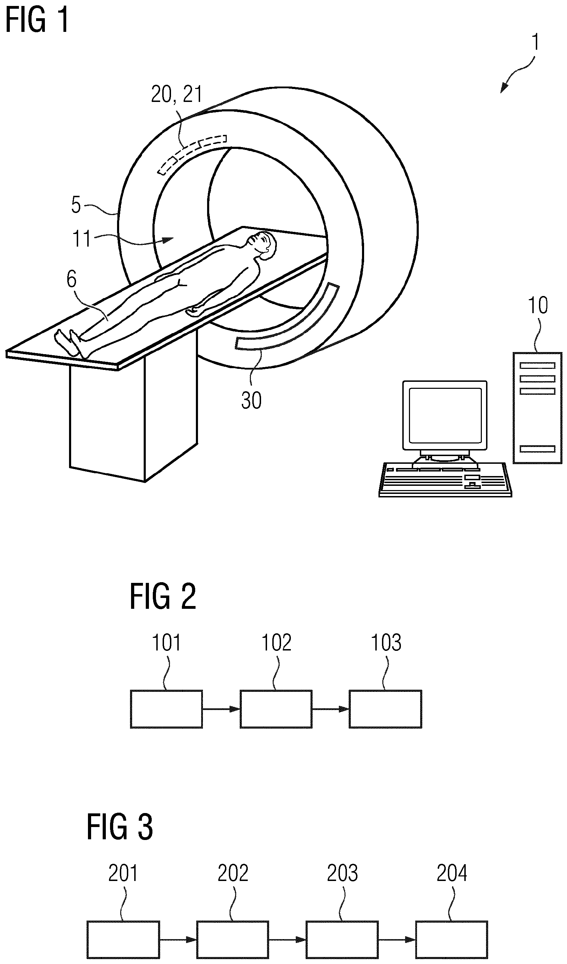

Figure 1 shows a computed tomography (CT) scanning system 1 with a gantry 5 according to an embodiment of the invention. Inside the gantry 5, there is an X-ray generating part 20 having at least one X-ray source 21 and an X-ray detecting part 30. Optionally, the X-ray detecting part 30 may comprise at least one photon counting detector that is configured to acquire X-ray data by differentiating between at least two X-ray energy levels of incoming X-rays by applying at least two energy thresholds. The X-ray generating part 20 and the X-ray detecting part 30 are rotatable around a scan region 11. The computed tomography scanning system 1 may be controlled by a control unit 10. The X-ray generating part 20 is capable of creating X-rays 23 via the at least one X-ray source 21 and sending the X-rays 23 into any part of the scan region 11. The X-ray detecting part 30 is configured to detect X-rays 23 generated by the X-ray generating part 20. During a scan in which the X-ray detecting part 30 is rotated around the scan region 11, the X-ray detecting part is configured to dynamically adjust the generated X-rays 23 such that at multiple rotational positions, X-rays 23 are selectively sent into a partial region and/or such that X-rays 23 of different intensity are sent into different partial regions.

Figure 2 shows a flow diagram of a method for performing a CT scan on a subject 6 according to an embodiment of the invention. The method comprises a first step 101 of receiving or determining a position of a region of interest 12 within a scan region 11. In a further step 102, an X-ray generating part 20 having at least on X-ray source 21, is rotated around the scan region 11 and X-rays are sent into the scan region 11 that pass through the region of interest 12. Optionally, in this step 102, first-type X-rays 23 and second-type X-rays 23 may be sent into the scan region 11, such that the first-type X-rays 23 pass through the region of interest 12 and the second-type X-rays 23 do not pass through the region of interest and have a lower intensity than the first-type X-rays 23. In a further step 103, the X-rays 23 are detected by an X-ray detecting part 30 after they have passed the region of interest 12.

Figure 3 shows a flow diagram of a method for performing a CT scan on a subject 6 according to an embodiment of the invention. The method comprises a first step 201 of receiving or determining a position of a region of interest 12 within a scan region 11. In a further step 202, first-type X-rays 23 and second-type X-rays 23 are sent into the scan region 11, such that the first-type X-rays 23 pass through the region of interest 12 and the second-type X-rays 23 do not pass through the region of interest 12 and have a lower intensity than the first-type X-rays 23. In a further step 203, the X-rays 23 are detected by an X-ray detecting part 30 after they have passed the region of interest 12. In a further step 204, a computed tomography image based on the data created via detecting the first-type X-rays 23 and using the second-type X-rays 23 is reconstructed to avoid the occurrence of truncation artifacts in the reconstructed image.

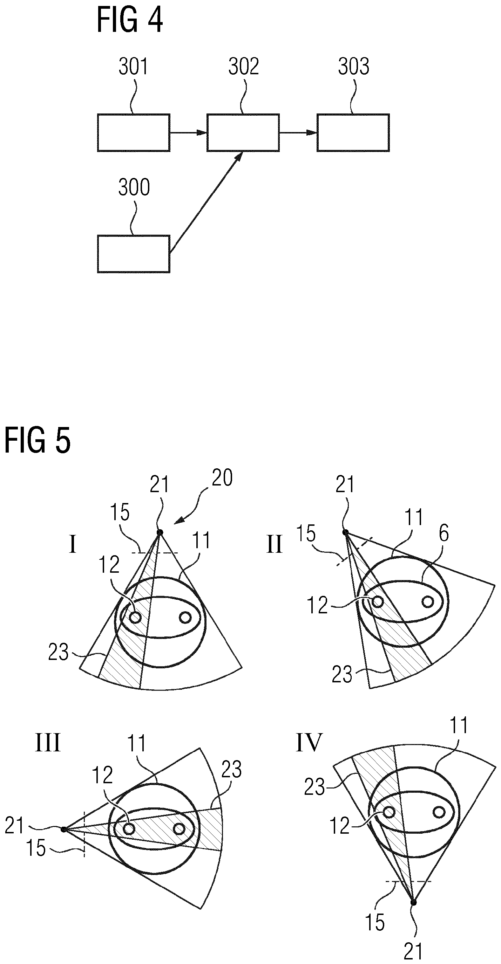

Figure 4 shows a flow diagram of a method for performing a fluence field modulated computed tomography scan on a subject 6 according to an embodiment of the invention. The method comprises a step 300 of determining an intensity profile according to a predetermined attenuation profile of the subject 6. In another step 301 a position of a region of interest 12 within a scan region 11 is determined. In a further step 302, first-type X-rays 23 and second-type X-rays 23, and optionally more types of X-rays 23, are sent into the scan region 11. The different types of X-rays 23 have different intensities. Namely, the different types of X-rays 23 sent into the scan region are modulated according to the intensity profile by controlling the intensity of X-rays 23 from individual X-ray sources 21 of an X-ray generating part. In a further step 303, the X-rays 23 are detected by an X-ray detecting part 30 after they have passed the region of interest 12.

Figure 5 shows several steps I-IV of the application of an X-ray generating part 20 according to an embodiment of the invention. In this embodiment, the X-ray generating part 20 comprises one X-ray source 21 and a collimator 15 is used to block some of the X-rays 23 generated by the X-ray source 21. During a CT scan, the X-ray generating part 20 is rotated around a scan region 11 and the collimator 15 is moved relative to the X-ray source 21 in order to block some of the X-rays that would not pass through a region of interest 12. As a result, in every angular rotation step of the X-ray generating part 20 that is shown, i.e. I-IV, only X-rays 23 that would pass through the region of interest 12 are not blocked by the collimator 15.

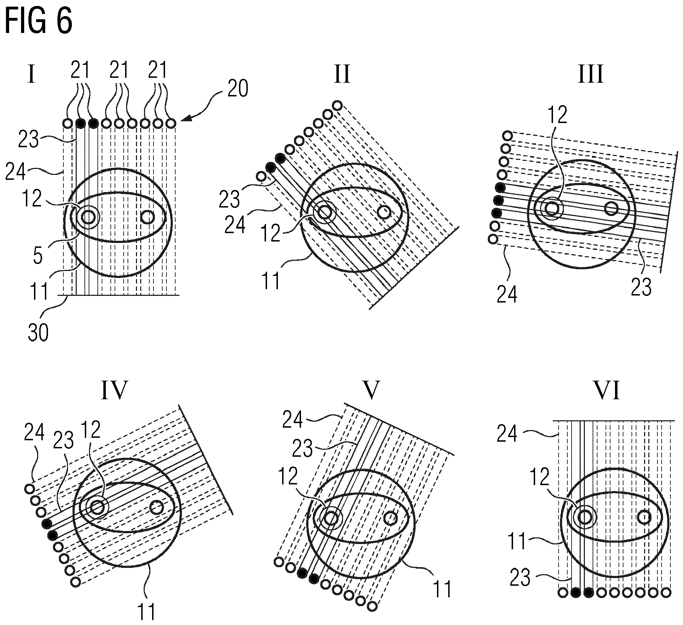

Figure 6 shows several steps I-VI of the application of an X-ray generating part 20 according to an embodiment of the invention. The X-ray generating part 20 comprises an X-ray-source array with a plurality of X-ray sources 21. The X-ray sources 21 are preferably field-emitter-based X-ray tubes, in particular carbon nano tubes. The X-ray generating part 20 is adapted to control the X-ray sources 21 independently of each other. In the different angular positions, I-VI, only those X-ray sources 21 are active, i.e. switched on, that create X-rays 23, depicted by full lines, that pass through a region of interest 12. Dashed lines 24 mark the theoretical pass, X-rays of currently switched off X-ray sources 21 would take. The X-ray sources 21 are dynamically switched on and off to ensure, that the region of interest 12 is covered fully by the X-rays 23, while other partial regions of the scan region 11 are not or are less illuminated than they would be if all X-ray sources were switched on all the time.

## Claims

1. A computed tomography scanning system (1) comprising a gantry (5) with
an X-ray generating part (20) having at least one X-ray source (21) and an X-ray detecting part (30),
wherein the X-ray generating part (20) and the X-ray detecting part (30) are rotatable around a scan region (11), the scan region (11) being dividable into partial regions,
wherein the X-ray generating part (20) is adapted such that it is capable of creating X-rays (23) via the at least one X-ray source (21) and sending the X-rays (23) into any part of the scan region (11) and
wherein the X-ray detecting part (30) is configured to detect X-rays (23) generated by the X-ray generating part (20),
wherein the X-ray generating part (20) is adapted to, during a scan in which the X-ray generating part (20) is rotated around the scan region (11), dynamically adjust the generated X-rays (23) such that, at multiple rotational positions, X-rays (23) are selectively sent into a partial region and/or such that X-rays (23) of different intensity are sent into different partial regions.

2. The computed tomography scanning system (1) according to claim 1,
wherein the system (1) is configured to receive a position of a region of interest (12) that is a partial region within the scan region (11),
wherein the system (1) is configured to have a scan mode in which the X-ray generating part (20) and the X-ray detecting part (30) are rotated around the scan region (11) and in which the X-ray generating part (20) is configured to send only X-rays (23) into the scan region (11) that pass through the region of interest (12),
or is configured to send only first-type X-rays (23) and second-type X-rays (23) into the scan region (11), wherein the first-type X-rays (23) pass through the region of interest (12), wherein the second-type X-rays (23) do not pass through the region of interest (12) and have a lower intensity than the first-type X-rays (23), in particular have no more than half the intensity of the first-type X-rays (23).

3. The computed tomography scanning system (1) according to claim 2,
wherein the system (1) is configured to perform at least a first scan protocol, in particular upon user activation, wherein the first scan protocol comprises
performing a planning scan covering the whole scan region (11),
prompting a user to choose the region of interest (12) from a visualization of the planning scan's image data or automatically determining the region of interest (12), and
consecutively performing a plurality of partial scans covering only part of the scan region (11) while covering the region of interest (12).

4. The computed tomography scanning system (1) according to any one of the preceding claims,
wherein the X-ray detecting part (30) comprises at least one photon counting detector, preferably a plurality of photon counting detectors,
wherein the at least one photon counting detector is in particular configured to acquire X-ray data by differentiating between at least two X-ray energy levels of incoming X-rays (23) by applying at least two energy thresholds.

5. The computed tomography scanning system (1) according to claim 3 and claim 4,
wherein the planning scan is a low-resolution scan,
wherein the partial scans are high resolution scans each having a higher resolution than the planning scan and wherein the photon counting detector is configured to apply at least two thresholds for each partial scan and for the low-resolution scan.

6. The computed tomography scanning system (1) according to any one of the preceding claims,
wherein the X-ray generating part (20) comprises a movable collimator (12) in front of the at least one X-ray source (21),
wherein the X-ray generating part (20) is configured to dynamically move the collimator (12) during a scan,
wherein the X-ray generating part (20) is rotated around the scan region (11) such that the X-rays (23) are dynamically adjusted, in particular such that X-rays (23) that would not pass through the partial region are blocked by the collimator (12).

7. The computed tomography scanning system (1) according to any one of the preceding claims,
wherein the X-ray generating part (20) is configured to dynamically change at least some of the pathways of the X-rays (23) relative to the current position of the X-ray generating part (20) during a rotation of the X-ray generating part (20).

8. The computed tomography scanning system (1) according to any one of the preceding claims,
wherein the X-ray generating part (20) comprises an X-ray-source array with a plurality of X-ray sources (21) including the at least one X-ray source (21),
wherein the X-ray generating part (20) is adapted to control the X-ray sources (21) independently of each other.

9. The computed tomography scanning system (1) according to claim 8,
wherein the X-ray generating part (20) is adapted to switch individual X-ray sources (21) on and off during a scan in which the X-ray generating part (20) is rotated around the scan region (11) such that only X-rays (23) are sent into the scan region (11) that pass through the partial region, in particular the region of interest (12).

10. The computed tomography scanning system (1) according to claim 9,
wherein the X-ray sources (21) of the source array are field-emitter-based X-ray tubes, in particular carbon nano tubes.

11. An X-ray generating part (20) for a gantry (5) of a computed tomography scanner, in particular according to any one the preceding claims, being rotatable around a scan region (11) and comprising
an X-ray-source array with a plurality of X-ray sources (21), the X-ray generating part (20) being adapted such that it is capable of creating X-rays (23) via the X-ray sources (21) and sending the X-rays (23) into any part of the scan region (11) and
wherein the X-ray generating part (20) is adapted to control the X-ray sources (21) independently of each other, in particular dynamically when the X-ray generating part (20) is rotated around a scan region (11).

12. A method for performing a computed tomography scan on a subject (6) in a scan region (11) with an X-ray generating part (20) having at least one X-ray source (21) and being rotatable around the scan region (11), in particular with a system (1) or X-ray generating part (20) according to any one of the preceding claims, comprising the following steps:
(a) receiving or determining a position of a partial region within the scan region (11), in particular a region of interest (12);
(b) rotating the X-ray generating part (20) around the scan region (11) and sending only X-rays (23) into the scan region (11) that pass through the partial region, or
sending first-type X-rays (23) and second-type X-rays (23) into the scan region (11), wherein the first-type X-rays (23) pass through the partial region wherein the second-type X-rays (23) do not pass through the partial region and have a lower intensity than the first-type X-rays (23), in particular have no more than half the intensity of the first-type X-rays (23);
(c) detecting the X-rays (23) by an X-ray detecting part (30) after they have passed the partial region.

13. The method according to claims 12, comprising performing the second alternative of step b using the first-type X-rays (23) and second-type X-rays (23) and further comprising the following additional step:
(d) reconstructing a computed tomography image based on the data created via detecting the first-type X-rays (23) and using the second-type X-rays (23) to avoid the occurrence of truncation artifacts in the reconstructed image.

14. The method according to claim 12, adapted for performing a fluence field modulated computed tomography scan on the subject (6) comprising the following additional step:
- determining an intensity profile according to a predetermined attenuation profile of the subject (6); wherein, in step (b), the X-rays (23) sent into the scan region (11) are modulated according to the intensity profile by controlling the intensity of X-rays (23) from individual X-ray sources (21).

15. A computer program comprising instructions which, when the program is executed by a computed tomography system (1), in particular a system (1) according to any one of claims 1 to 10, cause the system (1) to carry out the steps of the method according to any one of claims 12 to 14.
